(19) Europäisches Patentamt / European Patent Office / Office européen des brevets

(11) EP 2 821 788 A1

(12) **EUROPÄISCHE PATENTANMELDUNG**

(43) Veröffentlichungstag:
**07.01.2015 Patentblatt 2015/02**

(21) Anmeldenummer: **14183208.9**

(22) Anmeldetag: **26.06.2006**

(51) Int Cl.:
***G01N 33/497*** *(2006.01)* ***A61K 51/00*** *(2006.01)*
***A61B 5/097*** *(2006.01)* ***A61B 5/083*** *(2006.01)*
***A61M 16/06*** *(2006.01)* ***A61B 5/00*** *(2006.01)*
***A61K 49/00*** *(2006.01)* ***A61K 51/04*** *(2006.01)*

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **25.06.2005 DE 102005028836**

(62) Dokumentnummer(n) der früheren Anmeldung(en) nach Art. 76 EPÜ:
**06761702.7 / 1 896 846**

(71) Anmelder:
- **Freie Universität Berlin**
  **14195 Berlin (DE)**
- **Charité - Universitätsmedizin Berlin**
  **10117 Berlin (DE)**

(72) Erfinder:
- **Stockmann, Martin**
  **14129 Berlin (DE)**
- **Riecke, Björn**
  **22299 Hamburg (DE)**

(74) Vertreter: **Sokolowski, Fabian**
**Patentanwälte**
**Maikowski & Ninnemann**
**Postfach 15 09 20**
**10671 Berlin (DE)**

Bemerkungen:
Diese Anmeldung ist am 02-09-2014 als Teilanmeldung zu der unter INID-Code 62 erwähnten Anmeldung eingereicht worden.

(54) **Analyseverfahren zur Bestimmung eines Organfunktionsparameters**

(57) Die Erfindung betrifft ein Analyseverfahren zur Bestimmung eines Organfunktionsparameters eines menschlichen oder tierischen Individuums durch Messung des $^{13}CO_2$-Gehalts in der Ausatemluft des Individuums. Dabei ist der Organfunktionsparameter die Leberfunktionskapazität und/oder die Mikrozirkulation eines Substrates in der Leber, wobei die Leberfunktionskapazität durch Bestimmung der maximalen Umsatzgeschwindigkeit des Substrats im Körper des Individuums ermittelt wird und wobei die Mikrozirkulation durch Bestimmung der Anflutungszeit ermittelt wird. Die Anflutungszeit ist die Zeit zum Erreichen der maximalen Umsatzgeschwindigkeit $t_{vmax}$ des Substrates. Die maximale Umsatzgeschwindigkeit des Substrats im Körper des Individuums wird mittels einer Enzymkinetik nullter Ordnung durch eine Veränderung des gemessenen $^{13}CO_2$-Gehalts in der Ausatemluft des Individuums bestimmt wird. Die maximale Umsatzgeschwindigkeit und/oder die Anflutungszeit werden mittels einer Division durch das Körpergewicht auf das Körpergewicht des Individuums normiert.

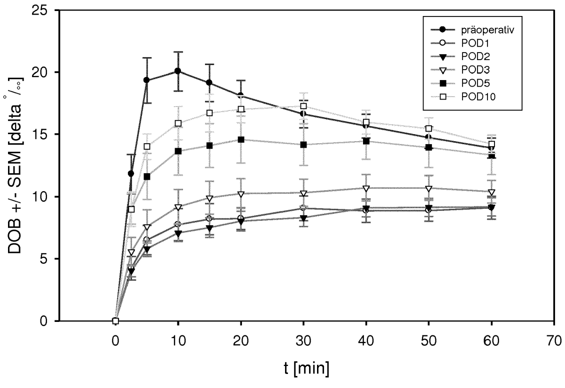


FIG 1



Printed by Jouve, 75001 PARIS (FR)

## Beschreibung

Die Erfindung betrifft ein Analyseverfahren zur Bestimmung eines Organfunktionsparameters nach dem Oberbegriff des Anspruchs 1.

Die Bestimmung eines Organfunktionsparameters, insbesondere die quantitative Bestimmung der Leberfunktion, ist in vielen Bereichen der Medizin von großer Bedeutung. Chronische Lebererkrankungen sind in Europa weit verbreitet, allein mit Hepatitis C sind 8,9 Millionen Menschen infiziert. Diese Individuen bzw. Patienten befinden sich mit fortschreitender Erkrankung in meist dauerhafter medizinischer Betreuung. In Therapie und Management von Patienten mit chronischen Lebererkrankungen kann durch Quantifizierung der Leberfunktion eine deutlich bessere Therapiesteuerung erfolgen und zur Fällung der richtigen Therapieentscheidungen ist die Einschätzung der Leberfunktion entscheidend.

Die Leberteilresektion ist ein gängiges Verfahren in der heutigen Chirurgie. Sie wird als Segmentresektion oder Hemihepatektomie entlang der anatomischen Grenzen durchgeführt. Ausgedehnte Eingriffe in dem parenchymatösen Organ wurden durch die Entwicklung der verschiedensten Operationstechniken ermöglicht. Die postoperative Morbidität und Mortalität durch Leberversagen aufgrund fehlender Leberfunktionskapazität bei vorgeschädigtem oder zu geringem Leberrestgewebe ist aber weiterhin ein bedeutendes Problem. Ein Großteil der operativen Eingriffe muss aber in vorgeschädigtem Lebergewebe, meist einer zirrhotisch umgebauten Leber, vorgenommen werden. Es ist daher notwendig, die funktionelle Leberkapazität eines Patienten schon vor der Leberteilresektion bestimmen zu können, um Patienten, die keine ausreichenden funktionellen Reserven ihres Lebergewebes mehr haben, nicht dem für sie hohen Operationsrisiko auszusetzen oder anderen Therapieverfahren zuzuführen.

In der Lebertransplantation kommt der Einschätzung der Leberfunktion besondere Bedeutung zu, da hier kurzfristig die Organfunktion eingeschätzt und eine schnelle Therapieentscheidung getroffen werden muss. Hier lässt sich außerdem in vielen klinischen Situationen schwer einschätzen, ob eine parenchymatöse Funktionsstörung vorliegt, oder ob andere Ursachen für die klinischen Symptome der Patienten verantwortlich sind. Zusammengefasst besteht daher ein erheblicher Bedarf, einen tatsächlich quantitativen Leberfunktionstest für die breite Anwendung in der Medizin anzubieten.

Es gibt deshalb weltweit Anstrengungen, einfache Tests zu entwickeln, die es ermöglichen prognostische Aussagen bezüglich der funktionellen Reserven des Leberzellgewebes zu machen. Konventionelle Laborparameter sind sehr unsicher und deshalb hierfür ungeeignet. Sie sind nicht sensitiv genug, die komplexen biologischen Vorgänge im Hepatozyten (Biosynthese, Biotransformation, Katabolismus von Xenobiotika etc.) sowie deren Veränderungen bei Erkrankung verlässlich zu evaluieren.

Zusätzlich unterliegen sie einer Vielzahl äußerer Einflüsse und werden hierdurch verfälscht. Beispielsweise werden sie teilweise durch den therapeutischen Interventionsbedarf durch Substitution von Humanplasma, Gerinnungsfaktoren oder Albumin verfälscht und somit als Leberfunktionsparameter unbrauchbar. Eine Vielzahl verschiedener Leberfunktionstests ist in der Literatur beschrieben worden (Matsumoto, K., M. Suehiro, et al. (1987). "[13C]methacetin breath test for evaluation of liver damage." Dig Dis Sci 32 (4): 344-8, 1987; Brockmoller, J. and I. Roots (1994). "Assessment of liver metabolic function. Clinical implications." Clin Pharmacokinet 27 (3): 216-48).

Bisher ist es aber mit keinem Testverfahren gelungen, valide und tatsächlich quantitative Aussagen zur Leberfunktion zu treffen. Bisher konnte in allen Verfahren nur eine signifikante Differenzierung zwischen verschiedenen Krankheitsgruppen mit klinisch schon erkennbaren Zeichen erreicht werden. Daher wird in der klinischen Praxis kein Leberfunktionstest in der Routine-Diagnostik eingesetzt, da diese in der derzeitigen Genauigkeit keinen zusätzlichen klinischen Nutzen bringen.

Der bisher verwandte $^{13}C$-Methacetin-Atemtest mit einer ausschließlich oralen Gabe der Substanz ist eine Methode, die die Leberfunktionskapazität von gesunden Probanden und Patienten mit einer chronischen Hepatitis ohne Zirrhose und mit Zirrhose in den unterschiedlichen Child-Pugh-Stadien unterscheiden kann (Matsumoto, K., M. Suehiro, et al. (1987). "[13C]methacetin breath test for evaluation of liver damage." Dig Dis Sci 32 (4): 344-8, 1987), eine tatsächliche Quantifizierung aber nicht erlaubt.

Die Substanz Methacetin wird über das Enzym CYP1A2 in der Leber in einer schnell ablaufenden Einschrittreaktion zu Paracetamol demethyliert, wobei anschließend $CO_2$ entsteht. Durch $^{13}C$-Markierung der über die Etherbrücke gebundenen Methylgruppe kann dann $^{13}CO_2$ in der Ausatemluft gemessen werden. Die folgende Formel (I) gibt die Strukturformel von Methacetin wieder:

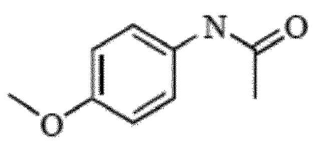

(I)

Mit den bisherigen Verfahren kann das Ziel einer tatsächlichen Quantifizierung mit einem individuellen Meßergebnis nicht erreicht werden. Dies hat zwei Ursachen:

1. Die Grundlage von Aussagen aus einem Atemtest ist, dass der zu evaluierende Schritt in der Kaskade der Vorgänge von Absorption und Metabolismus der geschwindigkeitsbestimmende Schritt sein muss. Bei den bisherigen Verfahren zur Evaluierung der Leberfunktion (orale Verabreichung der Testsubstanz) allerdings ist größtenteils die Resorption der geschwindigkeitsbestimmende Schritt, nicht die Umsetzung des Substrats in der Leber.

2. Um über ein Enzymsystem quantitative Aussagen treffen zu können - in dem vorliegenden Fall: um die maximale Leberfunktionsleistung, also die funktionelle Leberkapazität bestimmen zu können - muss das zu prüfende Enzymsystem wenigstens kurzfristig ausgelastet werden. Nur in diesem Fall läuft die Reaktion unabhängig von der Substratkonzentration ab.

Es ist also für eine tatsächliche Quantifizierung unabdingbar, den Substratüberschuss zu erreichen. Wird dies nicht erreicht, ist die Umsatzgeschwindigkeit direkt proportional zur und damit abhängig von der Substratkonzentration, die ihrerseits nichtlinear abfällt. Eine quantitative Aussage über die funktionelle Kapazität wird unmöglich. In sämtlichen Arbeiten mit oral eingesetzten Testsubstanzen konnte daher keine tatsächliche Quantifizierung erfolgen, da eine Enzymauslastung mit den bisherigen Verfahren nicht erreicht wird. Dies hat folgende Ursachen:

1. Bei oralem Einsatz muss Methacetin zunächst den Magen passieren und bis ins Duodenum und proximale Jejunum transportiert werden, um resorbiert werden zu können. Erst dann kann die Substanz über die Pfortader die Leber erreichen. Grundsätzlich kostet dieser Vorgang Zeit und bewirkt eine verzögerte und unvollständige Anflutung in der Leber. Dies ist äußerst variabel und wird von zahlreichen physiologischen und pathologischen Zuständen beeinflusst. Beispielsweise bei Leberzirrhose, bei der Leberfunktionstests zur Stadieneinteilung und zum Therapiemanagement eingesetzt werden könnten, ist die intestinale Passage und Resorption stark verändert (Castilla-Cortazar, I., J. Prieto, et al. (1997). "Impaired intestinal sugar transport in cirrhotic rats: correction by low doses of insulin-like growth factor I." Gastroenterology 113 (4): 1180-7) Auch im Verlauf nach abdominellen Operationen (z. B. Leberresektionen oder Lebertransplantation) ist durch eine Darmatonie (paralytischer Ileus) überhaupt keine sichere Aussage möglich.

2. Eine ausreichende Dosierung der Testsubstanz ist notwendig. Bei zu geringer Dosierung wie in den meisten Verfahren zur Durchführung des oralen Methacetin-Atemtests wird eine Auslastung des Enzymsystems per se nicht erreicht.

Ferner ist zu beachten, dass Methacetin in Wasser oder in einem wässrigen Puffer extrem schlecht löslich ist. Es kristallisiert aus einer gewöhnlichen wässrigen Lösung innerhalb von Stunden bis Tagen aus. Eine solche Lösung kann - wenn überhaupt - nur für orale Applikationen von Methacetin verwandt werden. Andere Applikationsformen sind nicht möglich.

Weiterhin wird in den bisherigen Verfahren die prozentuale Wiederfindungsrate der eingesetzten Dosis (Dosis%/h) sowie die kumulative Dosis zu bestimmten Zeitpunkten bzw. Zeitintervallen analysiert, um die Leberfunktion zu bestimmen. Die Berechnung der Dosis%/h definiert die umgesetzten Substratmengen nicht absolut und berücksichtigt ebenfalls das individuelle Körpergewicht des Patienten nicht. Eine Individualisierung und damit Normierung zur Einordnung der maximalen funktionellen Leberkapazität in ein Standardkollektiv kann hiermit nicht erfolgen.

Die bisherige Bestimmung der metabolisierten Kumulativdosis $D_{kum}$ über einen bestimmten Zeitraum ist bezüglich funktioneller Leberkapazität ebenso wenig aussagefähig. Für eine reliable (verlässliche) Aussage über die maximale Umsatzleistung des Enzymsystems über die Zeit müsste dieses hierbei über den gesamten Zeitraum ausgelastet sein. Dies ist aus den oben genannten Gründen nicht der Fall. Somit ist die derzeit verwendete Berechnung der Kumulativdosis zur Quantifizierung der funktionellen Leberkapazität unbrauchbar.

Zur Überführung der Ausatemluft eines Individuums in ein Messgerät bietet sich die Verwendung einer Atemmaske an, die auf das Gesicht des Individuums gesetzt wird. Dabei ist es von entscheidender Bedeutung für die anschließende verlässliche Durchführung eines Analyseverfahrens, dass die Ausatemluft sicher von der Einatemluft getrennt wird und darüber hinaus ein ungezwungenes Atmen des Individuums durch einen niedrigen Atemwegswiderstand der Atemmaske gewährleistet ist.

Verschiedene Typen von Atemmasken sind in der Medizin, in der Arbeitssicherheit oder auch beim Tauchen gebräuchlich. In der Medizin ist dies bei der Narkoseeinleitung, Narkoseführung oder auch zur Atemtherapie und nichtinvasiven Beatmung der Fall. Hierbei werden Masken mit guten Passform und dichten Sitz bevorzugt, wobei die erforderlichen Ventile außerhalb der Masken in den Schlauchsystemen oder den weiteren angeschlossenen Geräten eingebaut sind.

Bei Arbeitssicherheits-Masken oder auch Tauchmasken gibt es teilweise eingebaute Ventile, hier liegt der Fokus aber auf der Atemgaszuführung und dem sicheren Abschluss des Systems. Dadurch entstehen regelhaft hohe Atemwegswiderstände, so dass z.B. vor beruflichen Einsatz eines solchen Systems eine medizinische Eignungsprüfung notwendig ist.

Zur Analyse bestimmter Inhaltsstoffe in der Expriationsluft ist es notwendig, den Atemgasweg möglichst nah an der Entstehung der Ausatemsubstanzen, d.h. möglichst nah an den Alveolen der Lunge, zu separieren. Ansonsten kommt es zu einer Vermischung von Einatem- und Ausatemluft. Weiterhin darf es durch die Separierung nicht zu einer wesentlichen Erhöhung des Atemwegswiderstandes kommen, insbesondere nicht beim womöglich pulmonal kompromittierten Patienten. Speziell der Einatemwiderstand sollte sich nicht wesentlich erhöhen, da keine mechanische Unterstützung der Atemarbeit oder der Gaszufuhr erfolgen kann, wie dies z.B. bei der Narkose, Beatmung oder auch bei Tauch- oder Arbeitssicherheitsausrüstungen der Fall ist. Weiterhin ist es von wesentlicher Bedeutung, dass während der Analyse bekannt ist, ob die Atemmaske dicht auf dem Gesicht aufsitzt oder womöglich gerade abgesetzt wurde.

Der Erfindung liegt die Aufgabe zugrunde, ein verlässliches Analyseverfahren zur Bestimmung eines Organfunktionsparameters eines Individuums zu schaffen.

Diese Aufgabe wird durch ein Analyseverfahren zur Bestimmung eines Organfunktionsparameters eines menschlichen oder tierischen Individuums mit den Merkmalen des Anspruchs 1 gelöst. Gemäß diesem Analyseverfahren wird der $^{13}CO_2$-Gehalt in der Ausatemluft des Individuums gemessen, wobei das $^{13}CO_2$ im Körper des Individuums enzymatisch aus einem Substrat, das dem Individuum zuvor verabreicht wurde, gebildet wird und anschließend von dem Individuum ausgeatmet wird. Die Messung des $^{13}CO_2$-Gehalts in der Ausatemluft des Individuums erfolgt dabei mit einem entsprechenden Messgerät. Die maximale Umsatzgeschwindigkeit des Substrats im Körper des Individuums wird durch eine Veränderung des gemessenen $^{13}CO_2$-Gehalts in der Ausatemluft des Individuums mittels einer Enzymkinetik nullter Ordnung bestimmt. Das erfindungsgemäße Analyseverfahren geht also von einer enzymkinetischen Betrachtungsweise aus.

Vorzugsweise handelt es sich bei dem zu bestimmenden Organfunktionsparameter um die Leberfunktionskapazität und/oder die Mikrozirkulation in der Leber. Somit eignet sich das Analyseverfahren insbesondere zur Quantifizierung der funktionellen Leberkapazität des Individuums. Die Funktion der Leber ist als zentrales Stoffwechselorgan extrem komplex. Viele biochemische Synthese- und Abbauwege sind in der Leber vorhanden. Gemeinsam ist aber nahezu allen, dass sie über einen enzymatischen Stoffumsatz funktionieren.

Vorzugsweise wird das $^{13}CO_2/^{12}CO_2$-Verhältnis in der Ausatemluft des Individuums bestimmt. Dieser Wert kann als $^{13}CO_2/^{12}CO_2$ Ratio in die unten stehende Formel (1) eingesetzt werden.

In einer besonders bevorzugten Ausgestaltung der Erfindung erfolgt die Berechnung der maximalen Umsatzgeschwindigkeit (LiMAx) durch umgesetzte Substratmenge pro Zeit in μg/h/kg Körpergewicht zu variablen Zeitpunkten, an denen der Maximalwert erreicht wird, damit die tatsächliche Quantifizierung der maximalen funktionellen Leberkapazität erfolgen kann. Die Berechnung erfolgt nach folgender Formel (1), welche eine Enzymkinetik nullter Ordnung beschreibt:

$$\mathrm{LiMAx} = \frac{\left(\delta^{13}\mathrm{C}_{\mathrm{tmax}} - \delta^{13}\mathrm{C}_{\mathrm{t0}}\right)\cdot \mathrm{R}_{\mathrm{PDB}} \cdot \mathrm{P} \cdot \mathrm{M}}{\mathrm{KG}} \quad [\mu g/h/kg] \qquad (1)$$

Hierbei ist $\delta^{13}C$ die Differenz zwischen der $^{13}CO_2/^{12}CO_2$ Ratio der Probe und des Pee Dee Belmite (PDB) Standards in delta per mil, $R_{PDB}$ ist die $^{13}CO_2/^{12}CO_2$ Ratio des PDB-Standards (0,0112375), P ist die $CO_2$-Produktionsrate (300 mmol/h * Körperoberfläche in $m^2$), M ist das Molekulargewicht des Substrats und KG das aktuelle Körpergewicht des Individuums in kg.

In einer weiteren bevorzugten Ausgestaltung der Erfindung wird nicht das $^{13}CO_2/^{12}CO_2$-Verhältnis, sondern der absolute $^{13}CO_2$-Gehalt in der Ausatemluft des Individuums bestimmt. Dies ist beispielsweise mittels isotopenselektiver Infrarotspektroskopie möglich. In der Formel (1) wird dann direkt mit den absoluten $^{13}CO_2$-Volumen-Konzentrationen integriert über die Zeit statt der $^{13}CO_2/^{12}CO_2$-Verhältnisse gearbeitet, und es entfallen dadurch auch die Faktoren $R_{PDB}$ und P und damit die Abhängigkeit von der nur pauschal abgeschätzten $CO_2$-Produktionsrate. Die $^{13}CO_2$-Volumen-Konzentration stellt dabei die Konzentration des $^{13}CO_2$ in der gesamten Ausatemluft dar, das heißt, dass bei der bevorzugten Verwendung des $^{13}CO_2$-Gehalts in der Ausatemluft des Individuums neben der $^{13}CO_2$- Konzentration auch das Volumen des gesamten Atemgasstroms bestimmt wird. Dadurch erhält man ebenfalls die Stoffumsatzrate (μg/h/kg), das heißt, die umgesetzte Substratmenge pro Zeit normiert auf das Körpergewicht des Individuums.

Die Formel (1) vereinfacht sich bei Bestimmung der absoluten $^{13}CO_2$-Konzentration zur Formel (2):

$$\mathrm{LiMAx} = \frac{\int_{t=t_{max}}^{t=t_{max}+i} [^{13}\mathrm{CO}_2]dt \cdot \int_{t=t_{max}}^{t=t_{max}+i} \dot{V}dt \cdot \mathrm{M}}{\mathrm{KG}} \ [\mu g/h/kg] \qquad (2)$$

Hierbei ist [$^{13}CO_2$] die absolute Konzentration des $^{13}CO_2$ in der Ausatemluft des Individuums pro Volumeneinheit, $\dot{V}$ ist das Volumen pro Zeiteinheit, t ist die Zeit, $t_{max}$ der Zeitpunkt des maximalen Stoffumsatzes, i die entsprechend der Meßmethodik kleinste mögliche Zeitauflösung, M ist das Molekulargewicht des Substrats und KG ist das aktuelle Körpergewicht des Individuums in kg.

Zur Bestimmung des $^{13}CO_2$-Gehalts in der Ausatemluft wird vorzugsweise ein Infrarotspektrometer verwendet, da $^{13}CO_2$ eine gut separierbare Absorptionsbande im Infrarotbereich aufweist.

Bei NDIRS-Messgeräten (NDIRS = nicht dispersive Infrarotspektroskopie) wird der in der Ausatemluft enthaltene Wasserdampf vor der Messung vorteilhafterweise durch einen Feuchtetauscher, der vor das Messgerät geschaltet ist, entfernt, um unerwünschte Absorptionen des Wasserdampfs im Infrarotbereich zu vermeiden. Ein besonders geeigneter Feuchtetauscher ist beispielsweise ein Nafion-Feuchtetauscher. Andere Feuchtetauscher, die die Ausatemluft des Individuums effektiv zu trocknen vermögen, sind aber ebenso geeignet.

Bei einer isotopenselektiven Bestimmung des absoluten $^{13}CO_2$-Gehalts in der Ausatemluft eines Individuums entfällt diese Trocknung der zu analysierenden Ausatemluft vorzugsweise, da sich die Banden des Wasserdampfs nicht mit der oder den zu beobachtenden Bande(n) des $^{13}CO_2$ überlagern.

In einer bevorzugten Ausgestaltung der Erfindung wird neben der maximalen Umsatzgeschwindigkeit des Substrats im Körper des Individuums auch die Anflutungszeit, das heißt, die Zeit, die zum Erreichen der maximalen Umsatzgeschwindigkeit notwendig ist, bestimmt.

Die Anflutungszeit wird vorzugsweise zur Beurteilung der Mikrozirkulation in der Leber herangezogen, so dass Mikrozirkulationsstörungen erkennbar werden. Schnellstmögliche Anflutung durch insbesondere intravenöse Bolusinjektion und Substratüberschuss sowie den hohen first-pass-Effekt von Methacetin oder einem anderen Substrat vorausgesetzt, kann man die hepatische Mikrozirkulation besonders vorteilhaft beurteilen. Hierbei wird die Anflutungszeit bis zum Erreichen der maximalen Umsatzrate $t_{vmax}$ bestimmt, die sich bei Mikrozirkulationsstörungen verlängert, da in diesem Fall die Substratanflutung nicht in allen Bereichen der Leber gleichmäßig oder insgesamt verzögert ist. So kann eine Leberperfusion beurteilt werden.

Vorzugsweise werden die Mikrozirkulation und die Leberperfusion nicht nur isoliert beurteilt, sondern auch in ein Standardkollektiv eingeordnet. Dazu wird die Anflutungszeit unter Berücksichtung des Körpergewichts des Individuums mit einem Normalkollektiv normiert. Gleiches gilt auch für die maximale Umsatzgeschwindigkeit. Durch den Bezug auf das individuelle Körpergewicht werden die Elimination einer interindividuellen Variabilität und damit eine Normierung erreicht. Erst hiermit ist die Einordnung der individuellen funktionellen Leberkapazität in ein Vergleichskollektiv möglich. Mit dem erfindungsgemäßen Verfahren ist man in der Lage, nicht nur zwischen eingeschränkter Leberfunktion (z. B. bei manifester Leberzirrhose) und gesunder Leberleistung zu unterscheiden, sondern durch die schnelle und vollständige Auslastung des Enzymsystems können nunmehr sehr geringe Unterschiede in der maximalen Umsatzrate (funktionelle Leberkapazität) über einen weiten Messbereich festgestellt werden.

Um den Zeitpunkt der maximalen Umsatzgeschwindigkeit detektieren zu können, ist eine Analyse der $^{13}CO_2$/$^{12}CO_2$ Ratio bzw. des relativen oder absoluten $^{13}CO_2$-Gehalts in der Ausatemluft über die Zeit notwendig.

Daher werden vorzugsweise zu definierten Zeitpunkten Atemgasproben diskontinuierlich gewonnen und mit einem Atemgasanalysator (Messgerät) nach einer der oben beschriebenen Techniken analysiert. So können die Atemgasproben beispielsweise zu den Zeitpunkten 0 min und 2½, 5, 10, 15, 20, 30, 40, 50 und 60 min nach Applikation der Testsubstanz (Substrat) gewonnen werden. Dieses Messverfahren bezeichnet man auch als diskontinuierliche Offline-Messung. Die Messung der Atemgasproben, das heißt, der zu analysierenden Ausatemluft, kann dabei direkt im Anschluss an die Probengewinnung oder mit einer zeitlichen Verzögerung erfolgen. Das heißt, die gewonnenen Atemgasproben können vor einer Messung zwischengelagert werden, wenn beispielsweise gerade kein Messgerät zur Verwendung bereit steht.

Idealer- und bevorzugterweise wird eine kontinuierliche Analyse der Ausatemluft mit einem Atemgasanalysator als Messgerät durchgeführt (Online-Messung).

Dabei erstreckt sich die Messung vorzugsweise über ein Zeitintervall, in dem die Enzymkinetik abläuft bzw. dass das kürzeste Zeitintervall ist, innerhalb dessen eine sichere Bestimmung der Enzymkinetik erfolgt. Bei kranken Individuen ist eine Analyse während eines Zeitintervalls von rund 60 Minuten notwendig. Bei gesunden Individuen, bei denen bereits nach wenigen Minuten die maximale Umsatzgeschwindigkeit des Substrats in der Leber erreicht wird, kann das Analyseverfahren bereits nach Erreichen der maximalen Umsatzgeschwindigkeit, das heißt nach wenigen Minuten (beispielsweise 5 Minuten) abgebrochen werden.

Aus der kontinuierlichen Online-Messung des $^{13}CO_2$-Gehalts in der Ausatemluft des Individuums resultiert insbesondere ein großer Zeitvorteil bei der Untersuchung gesunder Individuen, denn bei der Offline-Messung werden zunächst die Atemproben gesammelt und anschließend analysiert, so dass ein vorzeitiger Abbrechen des Analyseverfahrens kaum möglich ist. Aber auch bei kranken Individuen resultiert noch immer ein deutlicher Zeitvorteil, da das Ergebnis der Messung sofort nach Abschluss der Messung vorliegt und ein Versand an ein Labor oder ein erneuter Bedienereingriff entfällt.

Durch eine Erhöhung der Zeitauflösung bei der kontinuierlichen Online-Messung gegenüber der diskontinuierlichen Offline-Messung werden mehr Datenpunkte gewonnen, was in einer höheren Präzision der Kurvenanalyse resultiert.

Da bei der kontinuierlichen Online-Messung keine Atemgasbeutel zu festgelegten Zeiten aufgeblasen werden müssen, entfällt die Bedienerabhängigkeit hinsichtlich der entsprechenden Zeitpunkte; die Datenpunkte können somit mit einem signifikant reduzierten Fehler einem Zeitpunkt der ablaufenden Enzymkinetik zugeordnet werden.

Die kontinuierliche Online-Messung wird auch deshalb bevorzugt, da so eine vollautomatische Messung möglich ist, insbesondere wenn durch geeignete Maßnahmen der Gaszufluss (d.h. der korrekte Sitz einer Atemmaske auf dem Gesicht des Individuums) sichergestellt wird. Vorzugsweise wird die Ausatemluft des Individuums mittels einer Atemmaske am Gesicht des Individuums gesammelt und von dort über einen Schlauch oder eine andere Verbindungsleitung in das Messgerät überführt, um anschließend das Analyseverfahren durchzuführen.

In einer besonders bevorzugten Ausgestaltung der Erfindung wird als Substrat $^{13}C$-markiertes Methacetin verwendet. Dieses $^{13}C$-Methacetin hat ein Molekulargewicht von 166,19 g/mol.

Bei der Umsetzung des $^{13}C$-Methacetin in dem Körper des Individuums wird ein leberspezifisches Enzymsystem getestet, das aber nicht in so großem Verhältnis vorkommt, dass eine Auslastung des Enzyms zu keinem Zeitpunkt erreichbar wäre. Geeignet ist daher das Cytochrom-p450-Isoenzym CYP1A2. Über CYP1A2 werden nur verhältnismäßig wenig Substanzen verstoffwechselt, so dass die Einfluss- und Störfaktoren gering sind. Dennoch ist es als repräsentativ für die Leberfunktion anzusehen.

Die Substanz Methacetin wird über CYP1A2 in einer schnell ablaufenden Einschrittreaktion zu Paracetamol demethyliert, wobei anschließend $CO_2$ entsteht. Durch den hohen first-pass-Effekt ist eine schnelle und vollständige Umsetzung gewährleistet. $^{13}C$-Methacetin ist daher bestens geeignet. Durch $^{13}C$-Markierung der über die Etherbrücke gebundenen Methylgruppe kann dann $^{13}CO_2$ in der Ausatemluft gemessen werden. Dies kann durch Analyse der Ausatemluft mit einem geeigneten Atemgasanalysator erfolgen. Hierzu geeignet sind beispielsweise die Verfahren der isotopenselektiven nichtdispersiven Infrarotspektroskopie oder der isotopenselektiven Massenspektrometrie. Mit beiden Verfahren wird als Messwert die Differenz zwischen der $^{13}CO_2/^{12}CO_2$ Ratio ($^{13}CO_2/^{12}CO_2$-Verhältnis) der Probe und des Pee Dee Belmite (PDB) Standards in delta per mil ($\delta^{13}C$) geliefert.

Es wird auch eine wässrige Methacetin-Lösung offenbart. Der pH-Wert dieser Lösung ist so eingestellt, dass die Lösung basisch ist, wobei ein pH-Wert von 7,5 bis 9,5 und insbesondere von 8,0 bis 8,5 besonders bevorzugt ist. So hat sich beispielsweise ein pH-Wert von 8,2 als besonders vorteilhaft erwiesen.

Zur besseren Lösung des Methacetins weist die Methacetin-Lösung vorzugsweise einen Lösungsvermittler auf.

Besonders vorteilhafte Eigenschaften hinsichtlich einer Lösungsvermittlung weist dabei Propylenglykol auf, wobei vorzugsweise bei einer Konzentration von 10 bis 100 mg/ml, noch bevorzugter bei 20 bis 50 mg/ml, besonders bevorzugt bei 25 bis 35 und ganz besonders bevorzugt bei 30 mg/ml des Propylenglykols dessen vorteilhafte lösungsvermittlende Eigenschaften zum Tragen kommen.

Die Methacetin-Lösung ist in einer bevorzugten Variante der Erfindung steril und/oder pyrogenfrei, so dass die Methacetin-Lösung einem menschlichen oder tierischen Individuum bzw. Patienten appliziert werden kann, ohne dass gesundheitliche Komplikationen befürchtet werden müssen.

Das Methacetin hat in der Methacetin-Lösung vorzugsweise eine Konzentration von 0,2 bis 0,6 % (w/v), besonders bevorzugt von 0,3 bis 0,5 % und ganz besonders bevorzugt von 0,4 %. Bei dieser Konzentration ist das Methacetin in der erfindungsgemäßen Methacetin-Lösung gut löslich. Bei hinsichtlich der Löslichkeit bevorzugten niedrigeren Konzentrationen steigt das Volumen der Methacetin-Lösung, die einem zu untersuchenden Individuum appliziert werden muss, signifikant an, was unerwünscht ist. Bei einer höheren Methacetin-Konzentration besteht hingegen die Gefahr, dass das Methacetin aus der Lösung ausfällt bzw. auskristallisiert.

Um die Methacetin-Lösung für die Durchführung von Atemtests zur Bestimmung von Organfunktionsparametern vorteilhaft einsetzten zu können, ist das gelöste Methacetin vorzugsweise mit dem Kohlenstoffisotop $^{13}C$ markiert. Diese Markierung ist vorzugsweise nur auf die Bereiche des Moleküls beschränkt sein, die bei einer Umsetzung im Körper eines Individuums als $CO_2$ freigesetzt werden. Dies ist die in der Formel (I) am linken Rand dargestellte Methylgruppe. Durch eine Beschränkung der $^{13}C$-Markierung auf diese Methylgruppe weisen andere Abbauprodukte des Methacetins als das $CO_2$ keine Markierung auf, so dass diese Abbauprodukte Messungen, die auf der Bestimmung des Gehalts des $^{13}CO_2$ basieren, unbeeinträchtigt lassen.

Die Erfindung bezieht sich auch auf eine Verwendung einer erfindungsgemäßen Methacetin-Lösung in einem Analyseverfahren zur Bestimmung eines Organfunktionsparameters eines menschlichen oder tierischen Individuums

nach einem der Ansprüche.

Ferner ist die Verwendung einer erfindungsgemäßen Methacetin-Lösung in einem analytischen oder diagnostischen Verfahren zur Bestimmung der dynamischen Verteilung des Methacetins in einem Organ eines Individuums mittels kernmagnetischer Resonanzspektroskopie bzw. Magnetresonanztherapie (NMR bzw. MRT) ein Teilaspekt der Erfindung. Da $^{13}C$ NMR-aktiv ist, bietet es sich an, die dynamische Verteilung des Methacetins beispielsweise in der Leber zu untersuchen, um so Rückschlüsse auf Leberschäden ziehen zu können. In einem derartigen Verfahren können mit verhältnismäßig hoher Zeitauflösung (im Minutenbereich) dynamische Vorgänge in der Leber untersucht werden. Leberbereiche, die einer Methacetin-Durchströmung nicht zugänglich sind, werden auch mit anderen Substanzen nur noch unzureichend oder gar nicht mehr versorgt, so dass auf diese Weise eine Korrelation zu (partiellen) Leberschäden gezogen werden kann.

Es wird auch ein Diagnoseverfahren zur Bestimmung eines Organfunktionsparameters eines menschlichen oder tierischen Individuums bzw. Patienten beschrieben. Gemäß diesem Verfahren erfolgt eine intravenöse Injektion einer erfindungsgemäßen $^{13}C$-markierten Methacetin-Lösung in den Körper des Individuums und anschließend eine Analyse des relativen und/oder absoluten $^{13}CO_2$-Gehalts in der Ausatemluft des Patienten gemäß einem erfindungsgemäßen Analyseverfahren, das bereits oben erläutert wurde.

Vorzugsweise ist der zu bestimmende Organfunktionsparameter die Leberfunktionskapazität und/oder die Mikrozirkulation in der Leber.

Die zur enzymkinetischen Auswertung erforderliche schnelle Substratanflutung wird dadurch gewährleistet, dass $^{13}C$-Methacetin intravenös im Bolus verabreicht wird. Durch die intravenöse Bolusinjektion wird eine unmittelbare und vollständige Substratanflutung in der Leber erreicht. Die Resorption der Testsubstanz $^{13}C$-Methacetin ist nicht mehr der geschwindigkeitsbestimmende Schritt. Mit der bevorzugten Dosierung von 2 mg Methacetin pro kg Körpergewicht wird auch bereits beim Lebergesunden durch einen kurzfristigen Substratüberschuss eine temporäre Enzymauslastung ermöglicht. Auf diese Weise wird die tatsächliche quantitative Aussagefähigkeit möglich, da eine Enzymkinetik nullter Ordnung erreicht wird und nur diese eine Aussage über die maximale Leistungsfähigkeit eines Enzymsystems zulässt.

Die Erfindung soll anhand der nachfolgenden Figuren und eines Ausführungsbeispiels näher erläutert werden, ohne dass diese Erläuterungen eine einschränkende Wirkung auf den Schutzbereich der Erfindung entfalten sollen.

Es zeigen:

Fig. 1 eine graphische Darstellung von Delta-over-baseline-Mittelwerten über der Zeit einer Leberfunktionsstudie an Patienten, die einer Leberteilresektion unterzogen wurden;

Fig. 2 eine graphische Darstellung der aus den Daten der Figur 1 berechneten maximalen funktionellen Leberkapazitäten (LiMAx) in Abhängigkeit von der Zeit; und

Fig. 3 eine graphische Darstellung der mittleren Anflutungszeit des Substrats $^{13}C$-Methacetin in der Leber in Abhängigkeit von der Zeit zur Bestimmung der Mikrozirkulation in der Leber;

Die Figuren 1 bis 3 sollen anhand des nachfolgenden Ausführungsbeispiels näher erläutert werden.

Beispiel 1

Im Rahmen einer prospektiven Studie wurde mit einem Verfahren, das im Nachfolgenden näher charakterisiert ist, die Leberfunktion vor und nach Leberteilresektionen bei mehreren Patienten evaluiert:

1. Verfahren und Mittel zur enzymkinetischen Quantifizierung der Leberfunktionskapazität und zur Beurteilung von Mikrozirkulationsstörungen der Leber, wobei

   a) eine spezielle intravenös applizierbare Gebrauchslösung von $^{13}C$-markierten-Methacetin intravenös in ausreichender Dosierung injiziert wird und hierdurch eine schnelle Anflutung der Substanz in der Leber mit Substratüberschuß am verstoffwechselnden Enzymsystem gewährleistet wird.

   b) durch Analyse des $^{13}CO_2/^{12}CO_2$ - Verhältnisses in der Ausatemluft über einen Zeitraum von einer Stunde nach Injektion die maximale Umsatzgeschwindigkeit des $^{13}C$-markierten Methacetins nach einer Enzymkinetik nullter Ordnung bestimmt wird.

   c) durch Normierung auf das individuelle Körpergewicht eine Vergleichbarkeit mit einem Normalkollektiv hergestellt wird.

d) durch Bestimmung der Anflutungszeit bis zum Erreichen der maximalen Umsatzgeschwindigkeit Mikrozirkulationsstörungen der Leber beurteilt werden können.

Dabei kann das $^{13}CO_2/^{12}CO_2$ - Verhältnis idealerweise kontinuierlich oder sonst zu den definierten Zeitpunkten 0 min und 2½, 5, 10, 15, 20, 30, 40, 50 und 60 min nach Injektion des $^{13}$C-markierten Methacetins bestimmt werden.

Die Figur 1 zeigt die der prospektiven Studie zugehörigen Kurven der Delta over baseline (DOB)-Mittelwerte des $^{13}CO_2/^{12}CO_2$-Verhältnisses in der Ausatemluft der Patienten zu den einzelnen Messzeitpunkten im zeitlichen Verlauf.

Präoperativ ist bei normaler maximaler funktioneller Leberkapazität ein Kurvenverlauf mit steilem Anstieg und erneutem Abfall nach Erreichen des Maximalwertes zu erkennen. Nach Leberteilresektion ändert sich am 1. postoperativen Tag (POD1) der Kurvenverlauf drastisch hin zu einer Sättigungskinetik bei deutlich reduzierter Maximalfunktion. Mit zunehmender Leberregeneration nach der Leberteilresektion ändert sich die Kurvenform wieder hin zu der präoperativen Kurve (POD3-10).

Die hieraus berechnete tatsächliche funktionelle Leberkapazität in $\mu$g/h/kg gibt die Figur 2 wieder. Am POD1 fällt die maximale funktionelle Leberkapazität (LiMAx) von 301 +/- 24 $\mu$g/h/kg auf 141 +/- 13 $\mu$g/h/kg ab, um sich dann langsam wieder auf einen Wert von 249 +/-17 $\mu$g/h/kg am 10. postoperativen Tag (POD10) zu regenerieren.

Die Analyse der Mikrozirkulation, welche in Figur 3 dargestellt ist, zeigt parallel nach der Leberteilresektion eine deutliche Störung mit einer Verlängerung der Anflutungszeit ($t_{vmax}$) von 11,9 +/- 2,17 min auf 53,5 +/- 7,51 min, die im sich im weiteren Verlauf wieder erholt.

## Patentansprüche

1. Analyseverfahren zur Bestimmung eines Organfunktionsparameters eines menschlichen oder tierischen Individuums durch Messung des $^{13}CO_2$-Gehalts in der Ausatemluft des Individuums, welchem $^{13}$C-markiertes Methacetin als Substrat derart verabreicht wurde, dass in der Leber wenigstens kurzfristig ein Substratüberschuss vorliegt, wobei die Ausatemluft des Individuums durch die Umsetzung des Substrats im Körper des Individuums $^{13}CO_2$ enthält, unter Verwendung eines Messgeräts,
**dadurch gekennzeichnet,**
**dass** der Organfunktionsparameter die Leberfunktionskapazität und/oder die Mikrozirkulation des Substrates in der Leber ist,
wobei die Leberfunktionskapazität durch Bestimmung der maximalen Umsatzgeschwindigkeit des Substrats im Körper des Individuums ermittelt wird,
wobei die Mikrozirkulation durch Bestimmung der Anflutungszeit ermittelt wird, wobei die Anflutungszeit die Zeit zum Erreichen der maximalen Umsatzgeschwindigkeit $t_{vmax}$ des Substrates ist,
wobei die maximale Umsatzgeschwindigkeit des Substrats im Körper des Individuums mittels einer Enzymkinetik nullter Ordnung durch eine Veränderung des gemessenen $^{13}CO_2$-Gehalts in der Ausatemluft des Individuums bestimmt wird
und wobei die maximale Umsatzgeschwindigkeit und/oder die Anflutungszeit mittels einer Division durch das Körpergewicht auf das Körpergewicht des Individuums normiert werden.

2. Analyseverfahren nach Anspruch 1, **dadurch gekennzeichnet, dass** der $^{13}CO_2$-Gehalt relativ zum $^{12}CO_2$-Gehalt in der Ausatemluft des Individuums bestimmt wird.

3. Analyseverfahren nach Anspruch 2, **dadurch gekennzeichnet, dass** die maximale Umsatzgeschwindigkeit des Substrats im Körper des Individuums mittels folgender Formel bestimmt wird:

$$\mathrm{LiMAx} = \frac{\left(\delta^{13}C_{tmax} - \delta^{13}C_{t0}\right) \cdot R_{PDB} \cdot P \cdot M}{KG},$$

in der $\delta^{13}$C die Differenz zwischen dem $^{13}CO_2/^{12}CO_2$-Verhältnis in der Ausatemluft des Individuums und dem des Pee-Dee-Belemnite-Standards in delta per mil, $R_{PDB}$ das $^{13}CO_2/^{12}CO_2$-Verhältnis des Pee-Dee-Belemnite-Standards, P die $CO_2$-Produktionsrate, M das Molekulargewicht des Substrats und KG das Körpergewicht des Individuums ist.

4. Analyseverfahren nach einem der vorhergehenden Ansprüche, **dadurch gekennzeichnet, dass** der absolute

$^{13}CO_2$-Gehalt in der Ausatemluft des Individuums bestimmt wird.

**5.** Analyseverfahren nach Anspruch 4, **dadurch gekennzeichnet, dass** die maximale Umsatzgeschwindigkeit des Substrats LiMAx im Körper des Individuums mittels folgender Formel bestimmt wird:

$$\mathrm{LiMAx} = \frac{\int_{t=t_{max}}^{t=t_{max}+i} [^{13}\mathrm{CO_2}]dt \cdot \int_{t=t_{max}}^{t=t_{max}+i} \dot{V} dt \cdot \mathrm{M}}{\mathrm{KG}},$$

in der [$^{13}CO_2$] die absolute $^{13}CO_2$-Konzentration pro Volumeneinheit, V das Volumen pro Zeiteinheit, t die Zeit, $t_{max}$ der Zeitpunkt des maximalen Stoffumsatzes, i die entsprechend der Meßmethodik kleinste mögliche Zeitauflösung, M das Molekulargewicht des Substrats und KG das Körpergewicht des Individuums ist.

**6.** Analyseverfahren nach einem der vorhergehenden Ansprüche, **dadurch gekennzeichnet, dass** die zu analysierende Ausatemluft diskontinuierlich zu festgelegten Zeitpunkten gesammelt und anschließend im Messgerät gemessen wird.

**7.** Analyseverfahren nach einem der Ansprüche 1 bis 5, **dadurch gekennzeichnet, dass** die zu analysierende Ausatemluft kontinuierlich im Messgerät gemessen wird.

**8.** Analyseverfahren nach einem der vorhergehenden Ansprüche, **dadurch gekennzeichnet, dass** sich die Messzeit nach der kürzesten Zeitspanne bemisst, die zur Bestimmung der Enzymkinetik, welche der Umsetzung des Substrats im Körper des Individuums zugrunde liegt, notwendig ist.

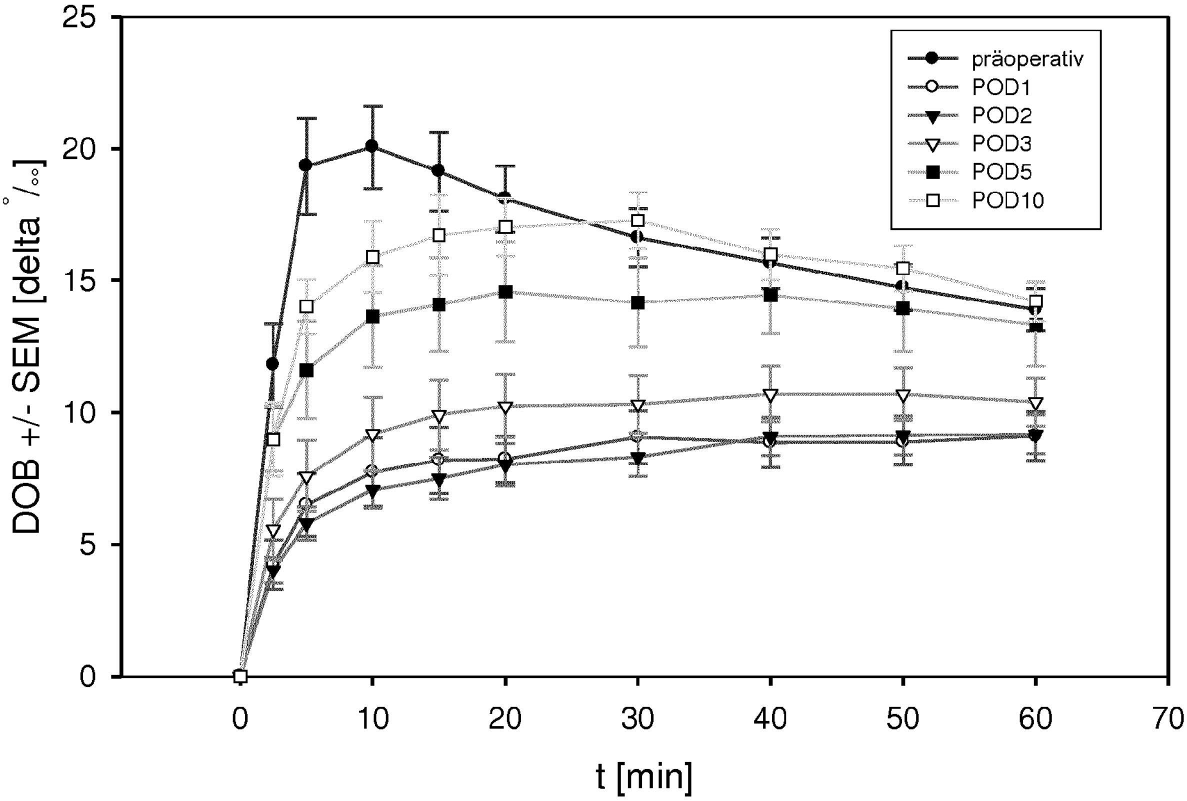
DOB +/- SEM [delta °/∞]
t [min]
präoperativ
POD1
POD2
POD3
POD5
POD10
0
5
10
15
20
25
0
10
20
30
40
50
60
70

FIG 1

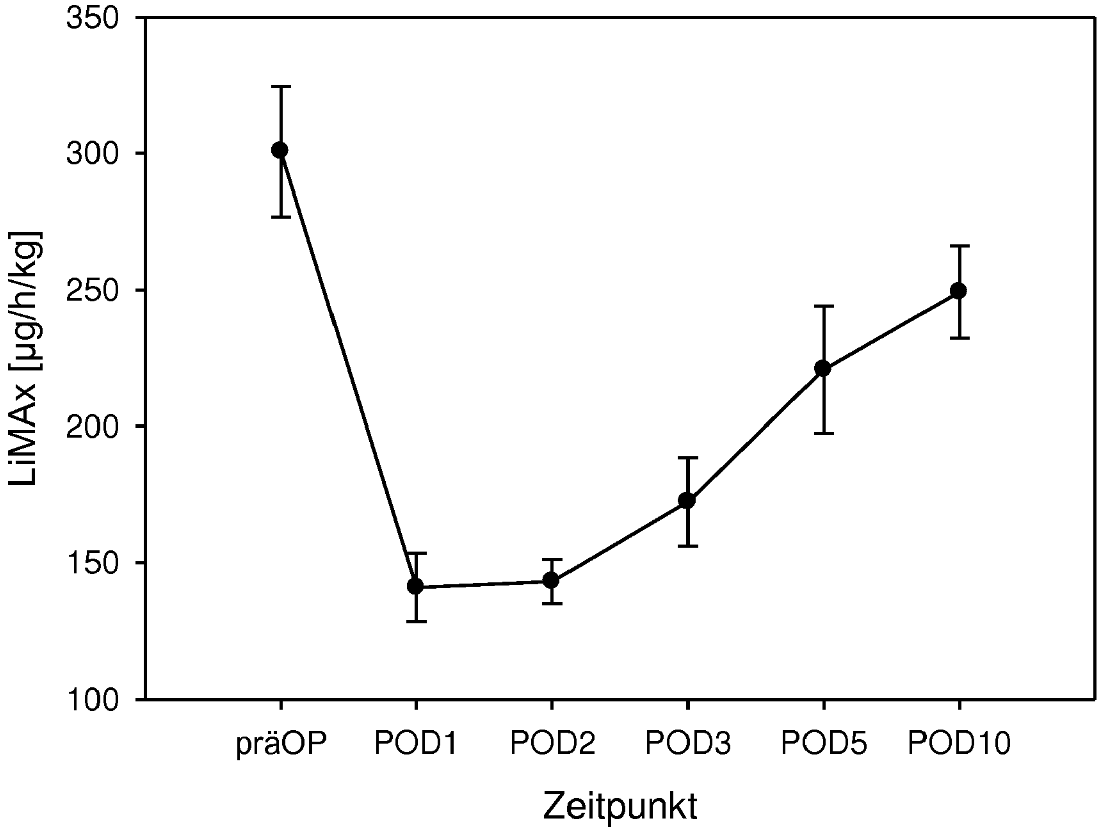
350
300
250
200
150
100
LiMAx [µg/h/kg]
präOP
POD1
POD2
POD3
POD5
POD10
Zeitpunkt

FIG 2

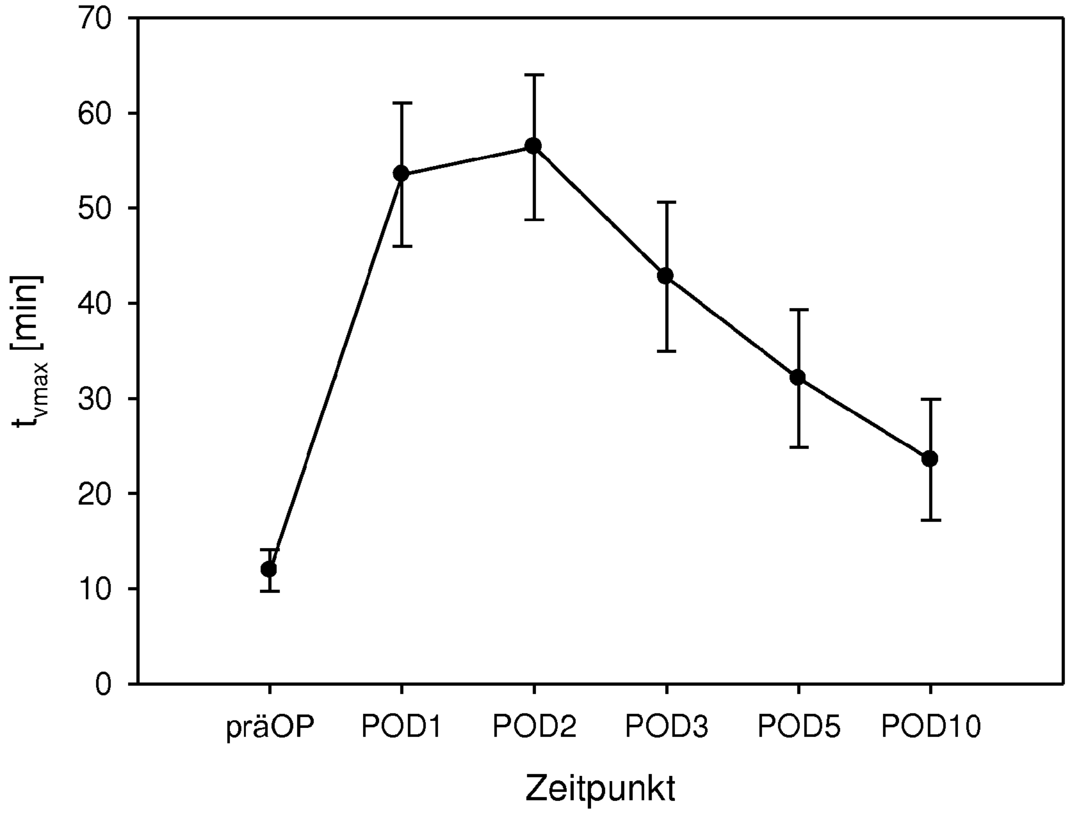
70
60
50
40
30
20
10
0
$t_{vmax}$ [min]
präOP
POD1
POD2
POD3
POD5
POD10
Zeitpunkt

FIG 3

Europäisches Patentamt
European Patent Office
Office européen des brevets

# EUROPÄISCHER RECHERCHENBERICHT

Nummer der Anmeldung
EP 14 18 3208

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | MATSUMOTO ET AL: "[13C]methacetin breath test for evaluation of liver damage", DIGESTIVE DISEASES AND SCIENCES, Bd. 32, Nr. 4, April 1987 (1987-04), Seiten 344-348, XP002400541, * Zusammenfassung * * Seite 345, linke Spalte, Zeile 16 - rechte Spalte, Zeile 9 * ----- | 1-8 | INV. G01N33/497 A61K51/00 A61B5/097 A61B5/083 A61M16/06 A61B5/00 A61K49/00 A61K51/04 |
| Y | BRADEN B ET AL: "13C-methacetin breath test as liver function test in patients with chronic hepatitis C virus infection", ALIMENTARY PHARMACOLOGY & THERAPEUTICS, Bd. 21, Januar 2005 (2005-01), Seiten 179-185, XP002400542, * Seite 181, linke Spalte, Absatz 4 - Seite 182, linke Spalte, Absatz 1 * ----- | 1,2 | |
| Y | EP 0 913 161 A (TOKYO GAS CO., LTD) 6. Mai 1999 (1999-05-06) * Seite 4, Absatz 52 - Seite 5, Absatz 54 * * Seite 6, Absatz 63 - Absatz 66 * * Seite 18, Absatz 238 * * Abbildung 1 * ----- | 4,7 | RECHERCHIERTE SACHGEBIETE (IPC) G01N A61K A61B A61M |
| A | LINGENFELSER TH ET AL: "Intravenous [<13>C]-methacetin breath test for evaluation of liver function in cirrhotic patients and healthy controls", GASTROENTEROLOGY, ELSEVIER, PHILADELPHIA, PA, Bd. 114, 15. April 1998 (1998-04-15), Seite A1291, XP027470142, ISSN: 0016-5085, DOI: 10.1016/S0016-5085(98)85241-4 [gefunden am 1998-04-15] * Zusammenfassung * ----- -/-- | 1-8 | |

Der vorliegende Recherchenbericht wurde für alle Patentansprüche erstellt

1

| Recherchenort | Abschlußdatum der Recherche | Prüfer |
|---|---|---|
| München | 2. Dezember 2014 | Lazar, Zala |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 14 18 3208

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | PAUWELS S ET AL: "Breath14CO2 after intravenous administration of [14C]aminopyrine in liver diseases", DIGESTIVE DISEASES AND SCIENCE, Bd. 27, Nr. 1, Januar 1982 (1982-01), Seiten 49-56, XP002400544, * Zusammenfassung * * Seite 49, linke Spalte, Zeile 1 - Seite 50, rechte Spalte, Zeile 26 * ----- | 3 | |
| Y | J VAUTHEY: "Body surface area and body weight predict total liver volume in Western adults", LIVER TRANSPLANTATION, Bd. 8, Nr. 3, 1. März 2002 (2002-03-01), Seiten 233-240, XP055112280, ISSN: 1527-6465, DOI: 10.1053/jlts.2002.31654 * Zusammenfassung * * Seite 233, Spalte 2, Absatz 1-3 * * Seite 235, Spalte 2, Absatz 2 * * Seite 239, Spalte 2, Absatz 2 * ----- | 1,2 | RECHERCHIERTE SACHGEBIETE (IPC) |
| Y | K. A. COPE: "Effects of ventilation on the collection of exhaled breath in humans", JOURNAL OF APPLIED PHYSIOLOGY, Bd. 96, Nr. 4, 1. April 2004 (2004-04-01), Seiten 1371-1379, XP055154619, ISSN: 8750-7587, DOI: 10.1152/japplphysiol.01034.2003 * Seite 1372, Spalte 1, Absatz 2 * * Seite 1373, Spalte 2, Absatz 2 * * Seite 1375, Spalte 1, Absatz 1 - Spalte 2, Absatz 2 * * Seite 1377, Spalte 1, Absatz 5 * ----- -/-- | 1-8 | |

1 Der vorliegende Recherchenbericht wurde für alle Patentansprüche erstellt

| Recherchenort | Abschlußdatum der Recherche | Prüfer |
|---|---|---|
| München | 2. Dezember 2014 | Lazar, Zala |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 14 18 3208

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| T | STOCKMANN M: "Wertigkeit eines neu entwickelten Verfahrens zur Bestimmung der Leberfunktion in der Leberchirurgie (LiMAx-Test)", HABILITATIONSSCHRIFT ERLANGUNG LEHRBEFÄHIGUNG FÜR DES FACH CHIRURGIE VORGELEGT DEM FAKULTÄTSRAT DER MEDIZINISCHEN FAKULTÄT CHARITÉ - UNIVERSITÄTSMEDIZIN BERLIN,, 19. Februar 2010 (2010-02-19), Seiten 1-48, XP007922910, * Seiten 33-36 * ----- | 1-8 | |
| A | WATKINS J B ET AL: "Diagnosis and differentiation of Fat Malabsorption in Children Using 13C-Labeled Lipids: Trioctanoin, Triolein, and Palmitic Acid Breath Tests", GASTROENTEROLOGY, ELSEVIER, PHILADELPHIA, PA, Bd. 82, Nr. 5, 1. Mai 1982 (1982-05-01), Seiten 911-917, XP002089943, ISSN: 0016-5085 * Seite 913, Spalte 1, Absatz 3-4 * ----- | 1-8 | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| München | 2. Dezember 2014 | Lazar, Zala |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 14 18 3208

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-12-2014

| Im Recherchenbericht angeführtes Patentdokument | Datum der Veröffentlichung | Mitglied(er) der Patentfamilie | Datum der Veröffentlichung |
|---|---|---|---|
| EP 0913161 A | 06-05-1999 | CA 2250485 A1 | 21-04-1999 |
| | | DE 69812532 D1 | 30-04-2003 |
| | | DE 69812532 T2 | 18-12-2003 |
| | | DE 69839314 T2 | 09-04-2009 |
| | | EP 0913161 A2 | 06-05-1999 |
| | | EP 1304124 A1 | 23-04-2003 |
| | | ES 2196501 T3 | 16-12-2003 |
| | | ES 2304472 T3 | 16-10-2008 |
| | | US 6509002 B1 | 21-01-2003 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **MATSUMOTO, K. ; M. SUEHIRO et al.** C]methacetin breath test for evaluation of liver damage. *Dig Dis Sci,* 1987, vol. 32 (4), 344-8
- **BROCKMOLLER, J. ; I. ROOTS.** Assessment of liver metabolic function. Clinical implications. *Clin Pharmacokinet,* 1994, vol. 27 (3), 216-48
- **CASTILLA-CORTAZAR, I. ; J. PRIETO et al.** Impaired intestinal sugar transport in cirrhotic rats: correction by low doses of insulin-like growth factor I. *Gastroenterology,* 1997, vol. 113 (4), 1180-7